# FASCICULE DE BREVET EUROPEEN

(11) **EP 1 556 410 B1**
(45) Date de publication et mention de la délivrance du brevet: **04.07.2012**
(21) Numéro de dépôt: 03767862.0
(22) Date de dépôt: 29.10.2003
(51) Int. Cl.: C07K 14/475

(54) **FRAGMENTS PEPTIDIQUES DU FACTEUR HARP INHIBANT L'ANGIOGENESE**
PEPTID FRAGMENTE DES HARP FAKTORS WELCHE DIE ANGIOGENESE INHIBIEREN
PEPTIDE FRAGMENTS OF THE HARP FACTOR INHIBITING ANGIOGENESIS

(30) Priorité: 30.10.2002 FR 0213621
(43) Date de publication de la demande: 27.07.2005
(73) Titulaire: CENTRE NATIONAL DE LA RECHERCHE SCIENTIFIQUE (CNRS), 75016 Paris (FR)
(72) Inventeur: COURTY, José, F-94440 Villecresnes (FR); BARRITAULT, Denis, F-75001 Paris (FR); PIERROT, Isabelle, F-94130 Nogent sur Marne (FR); DELBE, Jean, F-92410 Ville D'avray (FR); MILHIET, Pierre, F-34820 Teyran (FR)
(74) Mandataire: Colombet, Alain André
(86) Numéro de dépôt international: PCT/FR2003/003231
(87) Numéro de publication internationale: WO 2004/041859

(56) Documents cités:
- WO-A-02/083851
- HAMPTON B S ET AL: "Structural and functional characterization of full-length heparin-binding growth associated molecule." MOLECULAR BIOLOGY OF THE CELL, (1992 JAN) 3 (1) 85-93. , XP009029422
- BERNARD-PIERROT ISABELLE ET AL: "Dominant negative effectors of heparin affin regulatory peptide (HARP) angiogenic and transforming activities." JOURNAL OF BIOLOGICAL CHEMISTRY, (2002 AUG 30) 277 (35) 32071-7., XP002245580
- PAPADIMITRIOU E ET AL: "HARP induces angiogenesis in vivo and in vitro: implication of N or C terminal peptides." BIOCHEMICAL AND BIOPHYSICAL RESEARCH COMMUNICATIONS, (2001 MAR 23) 282 (1) 306-13., XP002245581
- PAPADIMITRIOU E ET AL: "Endothelial cell proliferation induced by HARP: Implication of N or C terminal peptides." BIOCHEMICAL AND BIOPHYSICAL RESEARCH COMMUNICATIONS, vol. 274, no. 1, 21 juillet 2000 (2000-07-21), pages 242-248, XP002245582 ISSN: 0006-291X

### LISTE DE SEQUENCES

<110> CNRS
<120> Fragments peptidiques du facteur HARP inhibant l'angiogénèse
<130> BET 03P0917
<140>
   <141>
<160> 7
<170> PatentIn Ver. 2.1
<210> 1
   <211> 136
   <212> PRT
   <213> Homo sapiens
<400> 1
   Gly Lys Lys Glu Lys Pro Glu Lys Lys Val Lys Lys Ser Asp Cys Gly
   1 5 10 15
   Glu Trp Gln Trp Ser Val Cys Val Pro Thr Ser Gly Asp Cys Gly Leu
   20 25 30
   Gly Thr Arg Glu Gly Thr Arg Thr Gly Ala Glu Cys Lys Gln Thr Met
   35 40 45
   Lys Thr Gln Arg Cys Lys Ile Pro Cys Asn Trp Lys Lys Gln Phe Gly
   50 55 60
   Ala Glu Cys Lys Tyr Gln Phe Gln Ala Trp Gly Glu Cys Asp Leu Asn
   65 70 75 80
   Thr Ala Leu Lys Thr Arg Thr Gly Ser Leu Lys Arg Ala Leu His Asn
   85 90 95
   Ala Glu Cys Gln Lys Thr Val Thr Ile Ser Lys Pro Cys Gly Lys Leu
   100 105 110
   Thr Lys Pro Lys Pro Gln Ala Glu Ser Lys Lys Lys Lys Lys Glu Gly
   115 120 125
   Lys Lys Gln Glu Lys Met Leu Asp
   130 135
<210> 2
   <211> 28
   <212> PRT
   <213> Homo sapiens
<400> 2
   Ser Asp Cys Gly Glu Trp Gln Trp Ser Val Cys Val Pro Thr Ser Gly
   1 5 10 15
   Asp Cys Gly Leu Gly Thr Arg Glu Gly Thr Arg Thr
   20 25
<210> 3
   <211> 33
   <212> PRT
   <213> Homo sapiens
<400> 3
   Ala Glu Cys Lys Tyr Gln Phe Gln Ala Trp Gly Glu Cys Asp Leu Asn
   1 5 10 15
   Thr Ala Leu Lys Thr Arg Thr Gly Ser Leu Lys Arg Ala Leu His Asn
   20 25 30
   Ala
<210> 4
   <211> 26
   <212> PRT
   <213> Homo sapiens
<400> 4
   Lys Leu Thr Lys Pro Lys Pro Gln Ala Glu Ser Lys Lys Lys Lys Lys
   1               5                   10                  15
   Glu Gly Lys Lys Gln Glu Lys Met Leu Asp
               20                  25
<210> 5
   <211> 84
   <212> ADN
   <213> Homo sapiens
<400> 5
   tctgactgtg gagaatggca gtggagtgtg tgtgtgccca ccagtggaga ctgtgggctg 60
   ggcacacggg agggcactcg gact                                        84
<210> 6
   <211> 93
   <212> ADN
   <213> Homo sapiens
<400> 6
   tgcaaatacc agttccaggc ctggggagaa tgtgacctga acacagccct gaagaccaga 60
   actggaagtc tgaagcgagc cctgcacaat gcc                              93
<210> 7
   <211> 75
   <212> ADN
   <213> Homo sapiens
<400> 7
   ctgaccaagc ccaaacctca agcagaatct aagaagaaga aaaaggaagg caagaaacag 60
   gagaagatgc tggat                                                  75

## Description

La présente invention concerne des fragments peptidiques de la protéine HARP inhibant l'angiogénèse.

La thérapie actuelle du cancer repose sur la radiothérapie, la chirurgie, parfois très invalidante et/ou l'utilisation de drogues anti-cancéreuses bloquant les mitoses et qui peuvent être très agressives limitant parfois leurs emplois. Il n'existe pas actuellement de thérapie universelle contre cette pathologie. Le rôle de l'angiogénèse dans la croissance tumorale a fait l'objet d'intenses recherches et il est maintenant admis par toute la communauté scientifique que la croissance tumorale ne peut se faire sans angiogénèse. Ce mécanisme qui est défini comme un processus dynamique induit par un certain nombre de facteurs angiogènes dont les principaux sont : le "vascular endothelial growth factor" (VEGF), le "fibroblast growth factor" (FGF) ou l'"heparin affin regulatory peptide" (HARP) qui sont dans plusieurs cas sur-exprimés par les tumeurs elles-mêmes. Puisqu'une tumeur ne peut se développer sans néo-vascularisation, la suppression ou l'inhibition des facteurs angiogènes doit conduire à une régression de la croissance tumorale et ceci quel que soit le type de tumeur. Aujourd'hui, plusieurs sociétés développent dans le traitement du cancer ou des maladies prolifératives, une stratégie anti-angiogénèse basée par exemple soit sur des inhibiteurs de facteurs angiogènes (ou de leur récepteurs quand ceux-ci sont identifiés), soit en induisant des micro thromboses vasculaires en utilisant la cellule endothéliale comme ancrage de catalyseurs de la thrombose, soit encore en utilisant des agents peptidiques qui inhibent l'angiogénèse par des mécanismes pas toujours identifiés. Ces approches n'ont pas encore débouché sur des résultats clairs et il apparaît que les inhibiteurs basés sur le blocage d'une seule voie de l'angiogénèse induisent des effets rebonds aggravants. Ces résultats conduisent actuellement ces sociétés à proposer des cocktails d'inhibiteurs permettant d'espérer une destruction radicale et simultanée des vaisseaux et de cellules tumorales.

Le rôle angiogène du facteur HARP a été montré au travers d'un ensemble d'expérimentations réalisées *in vitro* et *in vivo* (Papadimitriou et al. 2001 ; Papadimitriou et al., 2000). Ainsi dans un modèle *in vitro* dans lequel des cellules endothéliales sont ensemencées sur un gel de collagène, il a été montré que l'HARP est capable d'induire la formation de pseudo-capillaires mimant ainsi les premières étapes de l'angiogenèse, c'est-à-dire l'activation des cellules endothéliales et leur migration à travers une matrice extracellulaire partiellement détruite. Renforçant cette observation, un peptide synthétique de 43 acides aminés correspondant à une partie du domaine C-terminal de l'HARP est capable de stimuler la sécrétion de l'activateur du plasminogène par des cellules endothéliales d'aorte de boeuf (ABAE) et d'inhiber la sécrétion de son inhibiteur PAI-1 (Kojima et al., 1995a ; Kojima et al., 1995b). Cet activateur induit le clivage du plasminogène en plasmine, une protéase jouant un rôle clef dans la dégradation de la matrice extracellulaire. L'HARP partage cette propriété avec une autre protéine se liant à l'héparine (Matsubara et al., 1990) : la protéine Midkine (MK) présentant 50% d'homologie de séquence en acides aminés avec laquelle elle constitue une nouvelle famille d'HBGF (Tsutsui et al., 1991).

Le rôle joué par l'HARP dans l'angiogénèse tumorale a également été souligné par l'équipe de Roy Bicknell. Ainsi, la surexpression d'HARP dans des cellules de carcinome mammaire MCF7 injectées à des souris "nude" entraîne une augmentation de la taille de la tumeur par rapport à celles obtenues sans surexpression. L'augmentation de la taille de la tumeur est reliée à la densité vasculaire et à une multiplication des cellules endothéliales (Choudhuri et al., 1997). La prolifération des cellules endothéliales est une autre étape clef de l'angiogénèse dans laquelle l'HARP pourrait jouer un rôle crucial. En effet, dès 1991, il a été montré que l'HARP stimulait la prolifération des cellules endothéliales *in vitro* (Courty et al., 1991). Cette activité mitogène a pu être mise en évidence dans d'autres modèles : HARP stimule *in vitro* la formation en agar mou de colonies de cellules épithéliales SW13 non tumorigènes (Fang et al., 1992) et la surexpression de son ADNc dans les cellules NIH 3T3 (Chauhan et al., 1993) ou SW-13 (Fang et al., 1992) induit la formation d'une tumeur chez la souris "nude". Ce rôle mitogène est renforcé par l'expression de la molécule dans de nombreux cancers. En utilisant des tests de protection à la RNAse et/ou la technique Northern blot, les ARNm de l'HARP ont été détectés dans les lignées cellulaires issues de cancers du sein (T47 Dco, MDA-MB231, MDA-MB361, Hs-578T) (Fang et al., 1992), de l'ovaire (A1827, PA-1) (Riegel et al., 1994), de la prostate (PC-3) (Vacherot et al., 1999a) et du poumon (Jager et al., 1997).

*In vivo,* dans différents modèles tissulaires, la localisation de l'HARP est notamment associée aux cellules endothéliales des capillaires sanguins. De l'ARNm de la protéine HARP a été mis en évidence au niveau des cellules endothéliales appartenant aux capillaires sanguins de prostate et de glandes mammaires humaines (Vacherot et al., 1999a ; Ledoux et al., 1997). Dans le modèle utérin de rate, a également été mise en évidence une augmentation du taux d'ARNm et de la protéine HARP associée à la phase progestative du cycle. Ce résultat a été confirmé par l'injection de progestérone chez des rates ovariectomisées. La surexpression induite par la progestérone est détectable dans les cellules endothéliales capillaires de l'endomètre (Milhiet et al., 1998). La présence de la protéine HARP à la surface des cellules endothéliales a également été démontrée indirectement lors d'injections intraveineuses d'héparine chez l'homme (Novotny et al., 1993). Récemment, le groupe de Deuel a mis en évidence une augmentation de l'expression de l'ARNm de l'HARP dans les microvaisseaux en développement après une ischémie cérébrale chez le rat (Yeh et al., 1998). Les facteurs de croissance HARP et Midkine (MK) sont des molécules présentant 50% d'homologie et possédant des propriétés mitogènes sur des cellules épithéliales, fibroblastiques et endothéliales. L'expression de chacun de ces facteurs de croissance (ARNm, protéine) a été mise en évidence dans des tumeurs humaines d'origine variée (sein, poumon, ovaire, neuroblastome, estomac, colon), suggérant leur rôle potentiel au cours de la progression tumorale. Peu d'études cliniques ont évalué la coexpression de HARP et de MK et en particulier le taux sanguin de ces molécules angiogènes chez des patients porteurs de tumeurs. Récemment, a été développé un dosage immunologique pour ces deux molécules, basé sur leur affinité pour l'héparine. La sensibilité de la méthode est de 80 pg/ml pour l'HARP et de 40 pg/ml pour la MK (Stoica et al., 2001).

Le brevet U.S. 5,641,743 et le brevet U.S. 6,103,880 décrivent de manière générale la protéine HARP et l'utilisation de cette protéine pour stimuler l'angiogénèse.

La demande de brevet FR 2 799 465 divulgue un fragment de HARP à activité angiogène.

Il a en effet été montré que l'activité angiogène de HARP se retrouvait au travers d'un plus petit peptide. Une séquence consensuelle de 18 acides aminés issue de la séquence peptidique de HARP mais retrouvée également sur un grand nombre de facteurs angiogènes possédait ainsi par elle même une activité angiogène.

Comme la plupart des facteurs de croissance, le mécanisme d'action de HARP passe par une interaction avec un récepteur membranaire à activité tyrosine kinase de haute affinité (K_{D} = 50 pM) nommé "anaplastic lymphoma kinase" ou ALK (Stoica et al., 2001).

De plus, l'activité biologique de HARP, comme la majorité des HBGF, dépend également de son interaction avec des glycoaminoglycanes ou GAG, de type héparanes ou chondroïtine sulfates (Vacherot et al., 1999b).

Ainsi l'activité mitogène de l'HARP est potentialisée en présence d'héparine, d'héparane sulfate et de chondroïtine sulfate de type A et B. Un traitement cellulaire à l'héparinase abolit l'activité mitogène de l'HARP. Cette activité peut alors être restaurée en présence d'héparine. Une étude portant sur les relations entre la structure et la fonction de HARP a suggéré que la partie C-terminale de HARP correspondant aux résidus d'acides aminés 111-136 (numérotation faisant référence à la forme HARP₁₃₆) est directement impliqué dans l'induction de l'activité mitogène (Bernard-Pierrot et al., 2001) et dans son interaction avec le récepteur ALK. Il a par ailleurs été établi que le peptide 111-136 n'est ni mitogène, ni angiogène et que la molécule HARP dont les acides aminés 111-136 ont été délétés inhibe spécifiquement l'activité biologique de HARP en présentant des effets dominants négatifs vis-à-vis de HARP (Bemard-Pierrot et al. 2002).

Les inventeurs ont maintenant mis en évidence de manière surprenante que le facteur angiogène HARP contient des fragments peptidiques capables d'inhiber l'angiogénèse, ainsi que la croissance tumorale.

Il s'agit de tout ou partie des séquences d'acides aminés 13-39 et 65-97 localisés dans les domaines en feuillet beta de HARP.

Ces fragments sont donc particulièrement intéressants pour inhiber l'angiogénèse et la croissance tumorale, et peuvent, par conséquent, être mis à profit dans le traitement des désordres prolifératifs tels que le cancer, ou des pathologies pour lesquelles une angiogénèse excessive est observée, à savoir par exemple la rétinopathie diabétique.

Selon un mode de réalisation préféré de l'invention, il est proposé d'associer l'un et/ou l'autre de ces peptides à un peptide correspondant à tout ou partie de la séquence 111-136 de HARP. Cette association offre l'avantage d'éviter l'effet rebond" observé pour les autres techniques d'inhibition de certains facteurs angiogènes. En effet, il semble que l'inhibition *in vivo* d'un facteur angiogène soit compensée par l'activation d'un autre facteur angiogène.

Dans la présente Invention, les peptides 13-39 ou 65-97 agissent sur les récepteurs GAG, qui sont des récepteurs de basse affinité inhibant l'activité angiogène de HARP, tandis que le peptide 111-136 agit sur le récepteur ALK, de haute affinité.

Le blocage des sites de hautes affinités de HARP ainsi que ceux de basses affinités communs à d'autres facteurs angiogènes permet une thérapeutique particulièrement efficace dans le traitement des pathologies associées à un phénomène d'angiogénèse,

L'approche thérapeutique proposée consiste en une administration de peptides ou une administration d'acides nucléiques codant pour ces peptides. L'invention est définie par les revendications.

### Le facteur HARP et ses fragments

La présente invention concerne un peptide consistant en la séquence d'acides aminés choisie parmi : - la séquence 13-39 du facteur HARP (résidus 13 à 39 de SEQ ID NO: 1); et - la séquence 65-97 du facteur HARP (SEQ ID NO: 3). L'"heparin affin regulatory peptide" ou HARP (Courty et al., 1991) également appelée "pleiotrophin" (Wellstein et al., 1992) ou "heparin binding-growth associated molecule" ou HB-GAM (Rauvala et al., 1989) appartient à la famille des "heparin binding growth factors" (HBGF). La séquence peptidique humaine et le gène humain correspondant sont accessibles sur la base de données (Genbank n° X52946).

La protéine HARP est synthétisée sous la forme d'un précurseur de 168 acides aminés qui possède deux sites de clivage par la signal peptidase aboutissant à une protéine mature de 136 ou 139 acides aminés (HARP₁₃₆ ou HARP₁₃₉). Les formes matures HARP₁₃₆ et HARP₁₃₉ possèdent les mêmes activités biologiques *in vitro*, néanmoins l'activité spécifique d'induction de la prolifération cellulaire de la forme HARP₁₃₉ est meilleure que celle observée pour la forme HARP₁₃₆ (Bernard Pierrot et al., 2001).

La numérotation des acides aminés est donnée en fonction de la protéine HARP de 136 acides aminés.

La séquence peptidique humaine de HARP₁₃₆ est également présentée en séquence SEQ ID N° 1.

Dans le contexte de la présente invention, la protéine HARP ou ses fragments fait référence à la protéine humaine ou ses fragments, mais aussi à tout variant de la protéine chez d'autres espèces, notamment chez des mammifères de type bovins, rongeur, etc. Ainsi, les séquences de souris (Genbank N° AK017768 ou NM 008973), rat (Genbank N° NM017066), boeuf (Genbank N° X52945) sont disponibles. Sont également connus des variants chez Zebrafish (Genbank N° NM131070) et chez la drosophile (proteine miple Genbank N° NM 138178).

Il a été mis en évidence que des motifs d'homologie avec la thrombospondine de type TSR-1 (thrombospondin repeat type 1), présents dans les domaines en feuillet beta de HARP, notamment au niveau des acides aminés contenus dans les deux peptides 13-39 et 65-97, sont impliqués dans la fixation de l'HARP à l'héparine (Kilpelainen et al., 2000). Ces peptides se lient en fait aux récepteurs de basse affinité de type GAG.

De manière préférée, les fragments 13-39, 65-97 ou 111-136 sont les fragments tels que numérotés dans la séquence SEQ ID N° 1, à savoir les séquences ;

| | |
|---|---|
| 13-39 : | résidus 13 à 39 de SEQ ID N°1 |
| 65-97: | AECKYQFQAWGECDLNTAL.KTRTGSLKRALHNA (SEQ ID N° 3) |
| 111-136 : | KLTKPKPOAESKKKKKEGKKCEKMLD (SEO ID N° 4). |

Tout peptide « variant », « homologue », ou « dérivé » de ces peptides, qui présente la même activité biologique que ces peptides, fait également partie de l'invention.

Par *«activité biologique »,* on entend ici notamment une activité inhibitrice de l'angiogénèse. L'activité antiangiogène d'un composé peut aisément être évaluée *in vitro* ou *in vivo,* par l'homme du métier, notamment au moyen des tests suivants :
- *in vitro,* par mise en contact des peptides avec des cellules endothéliales sur du collagène, et observation de l'inhibition de la formation de capillaires ;
- *in vivo,* par injection de matrice de type Matrigel®, et observation de l'inhibition de la formation de capillaires au sein de la matrice ;
- ou encore en déposant le peptide sur une membrane choriantoallantoïdienne d'un oeuf (de poule par exemple), et en observant l'effet de ce peptide sur la vascularisation de cette membrane.

L'observation des cellules endothéliales (et par conséquent des capillaires) peut être réalisée en marquant spécifiquement ces cellules, par exemple au moyen de CD131 ou de lectine.

L'activité biologique des peptides 13-39 et 65-97 fait également référence à la capacité de ceux-ci de se fixer aux GAG de type héparanes ou chondroïtine sulfates. L'activité biologique du peptide 111-136 fait par ailleurs référence à la capacité de celui-ci de se fixer au récepteur ALK.

Les peptides « variants », « homologues», ou « dérivés » sont définis comme comprenant les séquences au moins 80 % identiques, préférence au moins 90 % identiques, voire au moins 95 % identiques, de la séquence de référence.

Ces peptides peuvent également être définis comme comprenant les séquences codées par une séquence d'acide nucléique hybridant avec la séquence de référence ou sa séquence complémentaire, dans des conditions stringentes d'hybridation.

Le terme "similaires" se réfère à la ressemblance parfaite ou identité entre les acides aminés comparés mais aussi à la ressemblance non parfaite que l'on qualifie de similitude. Cette recherche de similitudes dans une séquence polypeptidique prend en compte les substitutions conservatives qui sont des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires (tels que la glycine, l'alanine, la valine, la leucite, l'isoleucine, la proline, la phénylalanine, la méthionine, le tryptophane, et la cystéine).

Plus généralement, par *« séquence d'acides aminés variante, homologue, ou dérivée* », on entend donc toute séquence d'acides aminés qui diffère de la séquence de référence par substitution, délétion et/ou insertion d'un acide aminé ou plusieurs acides aminés, de préférence d'un nombre réduit d'acides aminés, notamment par substitution d'acides aminés naturels par des acides aminés non naturels ou pseudo-acides aminés à des positions telles que ces modifications ne portent pas significativement atteinte à l'activité biologique des peptides.

L'homologie est généralement déterminée en utilisant un logiciel d'analyse de séquence (par exemple, Séquence Analysis Software Package of the Genetics Computer Group, University of Wisconsin Biotechnology Center, 1710 University Avenue, Madison, WI 53705). Des séquences d'acides aminés similaires sont alignées pour obtenir le maximum de degré d'homologie (i.e. identité ou similitude, comme défini plus haut). A cette fin, il peut être nécessaire d'introduire de manière artificielle des espaces (« gaps ») dans la séquence. Une fois l'alignement optimal réalisé, le degré d'homologie est établi par enregistrement de toutes les positions pour lesquelles les acides aminés des deux séquences comparées sont identiques, par rapport au nombre total de positions.

De préférence, les peptides « variants », « homologues », ou « dérivés » sont de même longueur que les séquences de référence.

Le fragment consistant en les résidus 13 à 39 de SEQ ID N°1 et le fragment SEQ ID N° 3 contenant respectivement le motif 18-23 WQWSVC ou le motif 71-77 FQAWGEC sont particulièrement intéressants.

Conformément à l'invention, les peptides peuvent être par ailleurs modifiés chimiquement ou enzymatiquement pour améliorer leur stabilité ou leur biodisponibilité.

De manière non limitative, on peut par exemple modifier un ou plusieurs des acides aminés lysine (K) des peptides, notamment par :
- amidation : cette modification est simple à accomplir, la charge positive de la lysine étant substituée par des groupements hydrophobes (par exemple, acétyle ou phénylacétyle) ;
- amination : par formation d'amides secondaires à partir de l'amine primaire R = (CH₂)₄-NH₃⁺, par exemple en formant des groupements N-méthyle, N-allyle ou N-benzyle;
- ou encore par formation des groupements N-oxyde, N-nitroso, N-dialkyl phosphoryle, N-sulfényle, ou N-glycoside.

On peut par ailleurs aussi ou alternativement modifier un ou plusieurs acides aminés thréonine (T) et/ou sérine (S) des peptides, notamment en introduisant au niveau du groupe OH de la chaîne latérale de la thréonine et/ou de la sérine, un groupement ester ou éther. L'estérification, opération simple, peut être réalisée à l'aide d'un acide carboxylique, d'un anhydride, par pontages, etc, pour former par exemple des acétates ou des benzoates. L'éthérification, qui donne des composés plus stables, peut être effectuée à l'aide d'un alcool, d'un halogénure, etc, pour former par exemple un méthyl éther ou un O-glycoside.

On peut par ailleurs aussi ou alternativement modifier un ou plusieurs acides aminés glutamine (Q) par exemple par amidation, en formant des amines secondaires ou tertiaires, notamment avec des groupements de type méthyl, éthyl, fonctionnalisés ou non.

On peut par ailleurs aussi ou alternativement modifier un ou plusieurs acides aminés glutamate (E) et/ou aspartate (D), par exemple :
p ar estérification, pour former des méthyl esters substitués ou non, des éthyl esters, benzyl esters, des thiols (esters activés), etc,
p ar amidation, notamment pour former des groupements N,N diméthyle, nitroanilides, pyrrolidinyles, etc.

En revanche, il est préférable de ne pas modifier les acides aminés proline, qui participent à la structure secondaire des peptides, sachant en outre que les acides aminés G, A et M n'offrent généralement pas de possibilités de modification qui seraient manifestement intéressantes.

### Production des peptides

Les polypeptides utiles dans la présente invention peuvent être synthétisés par n'importe quelle méthode bien connue de l'homme du métier. Le peptide de l'invention peut par exemple être synthétisé par les techniques de la chimie de synthèse, telles que la synthèse de type Merrifield qui est avantageuse pour des raisons de pureté, de spécificité antigénique, d'absence de produits secondaires non désirés et pour sa facilité de production.

Un peptide recombinant peut également être produit par un procédé, dans lequel un vecteur contenant un acide nucléique codant pour le peptide est transféré dans une cellule hôte qui est mise en culture dans des conditions permettant l'expression du peptide correspondant.

Le peptide produit peut ensuite être récupéré et purifié.

Les procédés de purification utilisés sont connus de l'homme du métier. Le peptide recombinant obtenu peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono- ou polyclonaux spécifiques, etc.

L'acide nucléique utile pour la production de peptide recombinant peut notamment présenter les séquences suivantes :
- séquence codant pour le peptide 13-39 (SEQ ID N° 5)
- séquence codant pour le peptide 65-97 (SEQ ID N° 6)
- séquence codant pour le peptide 111-136 (SEQ ID N° 7)

Les acides nucléiques codant pour des peptides « variants », « homologues », ou « dérivés » des peptides de HARP décrits ci-dessus, font également partie de l'invention.

Ces acides nucléiques peuvent être définis comme comprenant :
i) des séquences similaires à au moins 70 %, de préférence au moins 80 %, de préférence au moins 90 %, voire au moins 95 % de la séquence SEQ ID n° 5, n° 6 ou n° 7 ; ou
ii) des séquences hybridant avec la séquence SEQ ID n° 5, n° 6 ou n° 7 ou sa séquence complémentaire, dans des conditions stringentes d'hybridation, ou
iii) des séquences codant pour le peptide de référence tel que défini précédemment.

De manière préférentielle, une telle séquence nucléotidique homologue hybride spécifiquement aux séquences complémentaires de la séquence SEQ ID n° 5, n° 6 ou n° 7 dans des conditions stringentes. Les paramètres définissant les conditions de stringence dépendent de la température à laquelle 50% des brins appariés se séparent (Tm).

Pour les séquences comprenant plus de 30 bases, Tm est définie par la relation : Tm=81,5+0,41(%G+C)+16,6Log(concentration en cations) - 0,63(%formamide) -(600/nombre de bases) (Sambrook et al., 1989).

Pour les séquences de longueur inférieure à 30 bases, Tm est définie par la relation : Tm= 4(G+C) + 2 (A+T).

Dans des conditions de stringence appropriées, auxquelles les séquences aspécifiques n'hybrident pas, la température d'hybridation peut être de préférence de 5 à 10°C en dessous de Tm, et les tampons d'hybridation utilisés sont de préférence des solutions de force ionique élevée telle qu'une solution 6xSSC par exemple.

Le terme "séquences similaires" employé plus haut se réfère à la ressemblance parfaite ou identité entre les nucléotides comparés mais aussi à la ressemblance non parfaite que l'on qualifie de similitude. Cette recherche de similitudes dans les séquences nucléiques distingue par exemple les purines et les pyrimidines.

Une séquence nucléotidique homologue inclut donc toute séquence nucléotidique qui diffère de la séquence SEQ ID n° 5, n° 6 ou n° 7 par mutation, insertion, délétion ou substitution d'une ou plusieurs bases, ou par la dégénérescence du code génétique, pour autant qu'elle code pour un peptide présentant l'activité biologique des fragments de HARP auxquels ils se réfèrent.

La séquence d'acide nucléique d'intérêt peut être insérée dans un vecteur d'expression, dans lequel elle est liée de manière opérante à un ou des élément(s) permettant son expression ou la régulation de son expression, tels que notamment des promoteurs, activateurs et/ou terminateurs de transcription.

Les signaux contrôlant l'expression des séquences nucléotidiques (promoteurs, activateurs, séquences de terminaison...) sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium.

Les vecteurs de clonage et/ou d'expression tels que décrits ci-dessus, contenant une séquence nucléotidique définie selon l'invention sont divulgués par la présente invention.

L'invention vise en outre les cellules hôtes transfectées, de manière transitoire ou stable, par ces vecteurs d'expression. Ces cellules peuvent être obtenues par l'introduction dans des cellules hôtes, procaryotes ou eucaryotes, d'une séquence nucléotidique insérée dans un vecteur tel que défini ci-dessus, puis la mise en culture desdites cellules dans des conditions permettant la réplication et/ou l'expression de la séquence nucléotidique transfectée.

Des exemples de cellules hôtes incluent notamment des cellules de mammifères, telles que les cellules COS-7, 293, MDCK, des cellules d'insectes telles que les cellules SF9, des bactéries telles que *E*. *coli* et des souches de levures telles que L40 et Y90.

### Thérapie génique

La présente invention divulgue la mise en oeuvre d'une thérapie génique. Il s'agit d'administrer à un patient un acide nucléique qui code pour le ou les peptides d'intérêt, dans des conditions telles que le ou les peptides sont exprimés *in vivo* par les cellules du patient dans lesquelles l'acide nucléique a été transféré.

L'acide nucléique administré comprend une séquence nucléotidique telle que définie ci-dessus, dans la partie « Production des peptides ».

Un tel acide nucléique peut être notamment sous la forme d'un vecteur d'ADN, par exemple un vecteur plasmidique. On peut administrer un ou plusieurs vecteurs, chaque vecteur pouvant porter une ou plusieurs séquence(s) codant pour au moins un des peptides d'intérêt, à savoir le peptide 13-39, 65-97 ou 111-136.

Selon un mode de réalisation préféré, on utilise un vecteur portant une séquence codant pour le peptide 13-39, un autre vecteur portant une séquence codant pour le peptide 65-97 et enfin éventuellement, un autre vecteur comprenant une séquence codant pour le peptide 111-136.

Le ou les vecteurs d'ADN peuvent être introduits *in vivo* par toute technique connue de l'homme du métier. En particulier, il est possible d'introduire le vecteur d'ADN *in vivo* sous une forme nue, c'est-à-dire sans l'aide d'un quelconque véhicule ou système qui faciliterait la transfection du vecteur dans les cellules (EP 465 529).

Un canon à gènes peut être également employé, par exemple en déposant l'ADN à la surface de particules « en or » et en projetant celles-ci de manière à ce que l'ADN pénètre à travers la peau d'un patient (Tang et al., 1992). Des injections au moyen d'un gel liquide sont également possibles pour transfecter à la fois peau, muscle, tissu gras et tissu mammaire (Furth et al., 1992 ; Robinson et al., 1997).

Des techniques de microinjection, électroporation, précipitation au phosphate de calcium, formulations à l'aide de nanocapsules ou de liposomes sont d'autres techniques disponibles.

Des nanoparticules en polyalkyl cyanoacrylate biodégradables sont particulièrement avantageuses. Dans le cas de liposomes, l'utilisation de lipides cationiques favorise l'encapsulation des acides nucléiques qui sont chargés négativement, et facilite la fusion avec les membranes cellulaires chargées négativement.

De manière alternative, le vecteur peut être sous la forme d'un virus recombinant comprenant, insérée dans son génome, une séquence d'acide nucléique qui code pour le ou lesdits peptide(s).

Le vecteur viral peut être de préférence choisi parmi un adénovirus, un rétrovirus, en particulier un lentivirus, ainsi qu'un virus adéno-associé (AAV), un virus de l'herpès, un cytomégalovirus (CMV), un virus de la vaccine, etc.

Des vecteurs lentivirus ont par exemple été décrits par Firat et al., (2002).

De manière avantageuse, le virus recombinant est un virus défectif. Le terme « virus défectif » désigne un virus incapable de se répliquer dans une cellule cible. Généralement, le génome des virus défectifs est dénué d'au moins les séquences nécessaires pour la réplication dudit virus dans la cellule infectée. Ces régions peuvent être soit éliminées, soit rendues non fonctionnelles ou encore substituées par d'autres séquences et en particulier par l'acide nucléique qui code pour le peptide d'intérêt. Néanmoins, de préférence, le virus défectif conserve malgré tout les séquences de son génome qui sont nécessaires pour l'encapsulation des particules virales.

Une administration ciblée de gènes est par exemple décrite dans la demande WO 95/28 494.

### Pathologies visées

Les peptides de l'invention présentent des propriétés d'inhibition de l'angiogénèse.

A ce titre, ces peptides ou les acides nucléiques exprimant ces peptides sont particulièrement utiles pour le traitement de diverses pathologies associées à une angiogénèse ou faisant intervenir des facteurs angiogènes.

Par « *traitement* », on entend traitement à titre curatif (visant à au moins soulager ou stopper le développement de la pathologie) ou prophylactique (visant à réduire le risque d'apparition de la pathologie).

Les pathologies visées incluent mais ne sont pas limitées à :
- Les désordres prolifératifs. On entend ici toute prolifération anormale de cellules, quelle soit bénigne ou maligne. Ces tumeurs incluent les mélanomes, les carcinomes, les sarcomes, rhabdomyosarcome, rétinoblastome, neuroblastome, ostéosarcome. Parmi les tumeurs solides, on peut citer notamment les tumeurs (primitives ou non) mammaires, ovariennes, pulmonaires, du col de l'utérus, du tractus digestif, notamment du colon, du système urologique, du foie, du pancréas, des os. Les tumeurs non solides sont également visées, à savoir notamment les leucémies ou les lymphomes.

Les désordres prolifératifs peuvent être traités à tout stade de la prolifération. Les peptides ou les acides nucléiques de l'invention sont en particulier utiles pour lutter contre le développement des métastases tumorales. Parmi les tumeurs bénignes, on peut enfin citer notamment les hémangiomes et les adénomes hépatocellulaires.

L'effet thérapeutique anti-angiogène des peptides de l'invention se concrétise dans ces applications notamment par un effet d'inhibition de la croissance tumorale.
- Les lésions oculaires, qui incluent notamment des pathologies de la rétine telles que la rétinopathie diabétique, la dégénérescence maculaire, l'occlusion de la veine ou de l'artère rénale, le glaucome, etc. Les peptides ou acides nucléiques de l'invention peuvent également être utiles pour traiter des lésions oculaires pouvant être la conséquence d'acte de chirurgie réparatrice comme la greffe de cornée.
- La polyarthrite rhumatoïde, qui est une maladie inflammatoire associée à une intense angiogénèse (Jackson et al., 1988).
- et les maladies de peau comme le psoriasis.

Les peptides de l'invention peuvent également être utiles comme composés abortifs, pour le contrôle des naissances, en bloquant l'angiogénèse utérine et donc l'implantation de l'embryon.

### Compositions pharmaceutiques

Un objet de l'invention est donc une composition pharmaceutique comprenant, en tant que principe actif, au moins un peptide 13-39, ou 65-97 d'HARP, ou un peptide dérivé de l'un de ceux-ci avec un ou plusieurs excipients pharmaceutiquement acceptables.

Un autre objet de l'invention est une composition pharmaceutique comprenant, en tant que principe actif, un acide nucléique qui comprend une séquence codant pour au moins un peptide 13-39, ou 65-97 d'HARP, ou un peptide dérivé de l'un de ceux-ci, liée de manière opérante à un ou des élément(s) permettant l'expression du peptide ou la régulation de son expression, avec un ou plusieurs excipients pharmaceutiquement acceptables.

Il est entendu que la séquence est éventuellement liée de manière opérante à un ou des élément(s) permettant l'expression du peptide ou la régulation de son expression.

Par *"excipient'* ou *"véhicule pharmaceutiquement* acceptable", on entend tout solvant, milieu de dispersion, agents retardant l'absorption etc, qui ne produisent pas de réaction secondaire, par exemple allergique, chez l'humain ou l'animal.

Selon un mode de réalisation préféré de l'invention, au moins l'un des peptides 13-39, 65-97, ou de leurs peptides dérivés, est associé au peptide 111-136 d'HARP ou à un peptide dérivé.

L'invention fournit donc également une composition pharmaceutique comprenant au moins un peptide 13-39, ou 65-97 d'HARP, ou un peptide dérivé de l'un de ceux-ci, en association avec un peptide 111-136 d'HARP ou un peptide dérivé, en présence d'un ou plusieurs excipients pharmaceutiquement acceptables.

Une composition pharmaceutique préférée comprend :
- le peptide consistant en les résidus 13-39 de SEQ ID N°1;
- le peptide 65-97 de séquence SEQ ID N° 3 ; et
- le peptide 111-136 de séquence SEQ ID N° 4.

De manière alternative ou combinée, l'invention fournit également une composition pharmaceutique comprenant un acide nucléique qui comprend une séquence codant pour un peptide 13-39 ou 65-97 d'HARP ou un peptide dérivé de run de ceux-ci, la séquence étant liée de manière opérante à un ou des élément(s) permettant son expression, en association avec un acide nucléique qui comprend une séquence codant pour un peptide 111-136 d'HARP ou un peptide dérivé, en présence d'un ou plusieurs excipients pharmaceutiquement acceptables.

Il est entendu que la ou les séquence(s) sont éventuellement liée(s) de manière opérante à un ou des élément(s) permettant l'expression du ou des peptide(s) ou la régulation de son(leur) expression.

Une composition préférée comprend :
- un acide nucléique codant pour le peptide consistant en les résidus 13-39 de SEQ ID N° 1;
- un acide nucléique codant pour le peptide 65-97 de séquence SEQ ID N°3;
- un acide nucléique codant pour le peptide 111-136 de séquence SEQ ID N° 4.

Les acides nucléiques peuvent être portés par un vecteur unique ou être sous la forme de vecteurs séparés.

Un autre mode de réalisation de l'invention inclut l'administration essentiellement simultanée de compositions séparées comprenant d'une part au moins un peptide 13-39, ou 65-97 de HARP, ou un peptide dérivé de l'un de ceux-ci ou un acide nucléique codant pour l'un de ces peptides et d'autre part un peptide 111-136 de HARP ou un peptide dérivé ou un acide nucléique codant pour ces peptides.

L'administration peut également être réalisée de manière séquentielle, au moyen de compositions séparées comprenant d'une part au moins un peptide 13-39, ou 65-97 de HARP, ou un peptide dérivé de l'un de ceux-ci ou encore un acide nucléique codant pour l'un de ces peptides et d'autre part un peptide 111-136 de HARP ou un peptide dérivé ou un acide nucléique codant pour ces peptides.

Au moins un peptide 13-39, ou 65-97 de HARP, ou un peptide dérivé de l'un de ceux-ci ou encore un acide nucléique codant pour l'un de ces peptides, et un peptide 111-136 de HARP ou un peptide dérivé ou un acide nucléique codant pour ces peptides, peuvent donc être individuellement administrés sous une forme pharmaceutiquement acceptable.

La posologie dépend naturellement de l'actif considéré, du mode d'administration, de l'indication thérapeutique, de l'âge du patient et de son état.

La dose de peptide est préférablement de 0,1 à 250 mg/kg par jour, de préférence de 1 à 100 mg/kg par jour.

La dose unitaire du composé de formule (I) comprend de préférence de 12,5 à 200 mg de ce composé.

Quand les compositions pharmaceutiques comprennent des acides nucléiques, les doses d'acide nucléique (séquence ou vecteur) à administrer sont adaptées également en fonction notamment du mode d'administration, de la pathologie ciblée ainsi que de la durée de traitement. Généralement, lorsque des virus recombinants sont utilisés, ceux-ci sont formulés et administrés sous la forme de doses d'environ 10⁴ à 10¹⁴ pfu/ml, de préférence 10⁶ à 10¹⁰ pfu/ml. Le terme « pfu » (unité formant plage) correspond à l'infectivité d'une solution virale, et peut être déterminée en infectant une culture cellulaire appropriée et en mesurant, généralement après 48 heures, le nombre de plages de cellules infectées. Les techniques pour déterminer le titre pfu d'une solution virale sont bien décrites par la littérature.

Les compositions pharmaceutiques de l'invention peuvent être formulées de manière à être administrées au patient par une unique voie ou par des voies différentes.

Lorsque l'administration par voie parentérale est envisagée, plus particulièrement par injection, les compositions de l'invention comprenant le ou les principes actifs se trouvent sous la forme de solutés et suspensions injectables conditionnées en ampoules ou flacons pour perfusion lente. L'injection peut notamment être réalisée par voie sous-cutanée, intramusculaire ou intraveineuse.

De manière préférée, en particulier dans le cas d'une tumeur solide, la composition pharmaceutique peut être injectée au site-même de l'angiogénèse.

Dans le cas d'une administration par voie orale, les compositions de l'invention se trouvent sous la forme de gélules, comprimés effervescents, comprimés nus ou enrobés, sachets, dragées, ampoules ou solutés buvables, microgranules ou formes à libération prolongée.

Les formes pour l'administration parentérale sont obtenues de façon conventionnelle par mélange du ou des principes actifs avec des tampons, des agents stabilisants, des conservateurs, des agents solubilisants, des agents isotoniques et des agents de mise en suspension. Conformément aux techniques connues, ces mélanges sont ensuite stérilisés puis conditionnés sous la forme d'injections intraveineuses.

A titre de tampon, l'homme du métier pourra utiliser des tampons à base de sels de phosphate organique.

Des exemples d'agents de mise en suspension englobent le méthylcellulose, l'hydroxyéthylcellulose, l'hydroxypropylcellulose, l'acacia et la carboxyméthylcellulose sodique.

En outre, des stabilisants utiles selon l'invention sont le sulfite de sodium et le métasulfite de sodium, tandis que l'on peut citer le p-hydroxybenzoate de sodium, l'acide sorbique, le crésol et le chlorocrésol en tant que conservateurs. Pour la préparation de solution ou de suspension orale, les principes actifs sont dissous ou mis en suspension dans un véhicule approprié avec un agent dispersant, un agent humectant, un agent de mise en suspension (par exemple la polyvinylpyrrolidone), un conservateur (tel que le méthylparaben ou le propylparaben), un agent correcteur de goût ou un colorant.

Pour la préparation de microcapsules, les principes actifs sont combinés à des diluants appropriés, des stabilisants appropriés, des agents favorisant la libération prolongée des substances actives ou tout autre type d'additif pour la formation d'un noyau central qui est ensuite revêtu d'un polymère approprié (par exemple une résine hydrosoluble ou une résine insoluble dans l'eau). Les techniques connues de l'homme du métier seront utilisées à cet effet.

Les microcapsules ainsi obtenues sont ensuite éventuellement formulées dans des unités de dosage appropriées.

Une administration par voie oculaire peut également être envisagée, en particulier dans le cas d'un traitement d'une lésion oculaire.

La composition pharmaceutique de l'invention se présente alors sous la forme d'une composition ophtalmique pour administration locale dans l'oeil, par exemple comme un collyre, ou une crème ophtalmique.

La composition ophtalmique peut être une solution aqueuse comprenant de l'eau distillée, une solution saline physiologique, dans laquelle les peptides de l'invention sont dissous. Un certain nombre d'additifs peut être incorporé dans la composition ophtalmique si nécessaire par exemple des agents de tampon, des agents assurant l'isotonicité avec les larmes, des conservateurs, des épaississants, des stabilisants, des antioxydants, des agents ajustant le pH, des agents chélatants, etc.

Les gouttes pour les yeux sont préparées par manipulation aseptique ou la stérilisation est réalisée à une étape appropriée de la préparation.

Les crèmes ophtalmiques peuvent être préparées de manière aseptique en mélangeant le principe actif à une base usuelle. Les bases pour les crèmes ophtalmiques sont par exemple la vaseline, le jelene 50 ou le plastibase, le macrogol, etc. Des surfactants peuvent être ajoutés pour augmenter l'hydrophilie. Des additifs tels que décrits ci-dessus, par exemple les conservateurs, peuvent être ajoutés si nécessaire.

En général, pour une application ophtalmique locale, un effet satisfaisant chez l'adulte est obtenu par l'administration d'une gouttelette dans l'oeil d'une préparation contenant de 0,001 à 10 %, de préférence 0,01 à 1 % poids/volume du composé de l'invention ou d'un sel pharmaceutiquement acceptable de celui-ci plusieurs fois, de préférence une à six fois par jour, et à chaque fois avec de préférence une à quatre gouttelettes par oeil, et dans le cas d'utilisation d'une crème ophtalmique, d'une préparation contenant 0,001 à 10 %, de préférence 0,01 à 1 % poids/volume du composé de l'invention ou d'un sel pharmaceutiquement acceptable de celui-ci, avec une application de préférence une à six fois par jour dans l'oeil.

Les peptides de l'invention peuvent également être formulés sous la forme de liposomes. Les liposomes sont formés à partir de phospholipides qui sont dispersés dans un milieu aqueux et forment spontanément des vésicules bicouches concentriques multilamellaires. Ces vésicules ont généralement un diamètre de 25 nm à 4 µm et peuvent être soniquées, conduisant à la formation de vésicules plus petites unilamellaires, de diamètre de 200 à 500 Å, contenant une solution aqueuse en leur coeur.

Les liposomes peuvent être particulièrement avantageux pour administrer le médicament à une cible cellulaire ou tissulaire précise. Pour cela, les lipides peuvent être couplés chimiquement à des molécules de ciblage, tels que des peptides de ciblage (par exemple hormones), ou des anticorps.

La présente invention a également pour objet au moins un peptide 13-39, ou 65-97 de HARP, ou un peptide dérivé de l'un de ceux-ci, éventuellement en association avec un peptide 111-136 de HARP ou un peptide dérivé, pour son utilisation dans le traitement d'une pathologie associée à une angiogénèse.

Les pathologies visées sont telles que mentionnées plus haut.

L'invention a en outre pour objet une méthode pour traiter une pathologie associée à une angiogénèse, chez un mammifère, plus particulièrement un humain, comprenant l'administration chez ce mammifère d'une quantité thérapeutiquement efficace d'au moins un peptide 13-39, ou 65-97 de HARP, ou un peptide dérivé de l'un de ceux-ci, éventuellement en association avec un peptide 111-136 de HARP ou un peptide dérivé de celui-ci.

Bien entendu, les peptides de l'invention ou acides nucléiques peuvent être utilisés seule ou en association avec tout autre principe actif. On peut notamment citer le peptide 150-183 de l'Amphotérine, qui inhibe la formation des métastases (Taguchi et al., 2000 ; Huttunen et al., 2002) et présente d'ailleurs des homologies avec le peptide d'HARP.

On peut également choisir d'employer une thérapie génique en exprimant également un acide nucléique codant pour le peptide 150-183 de l'amphotérine.

La présente invention a également pour objet l'utilisation d'un acide nucléique comprenant une séquence codant pour un peptide 13-39, ou 65-97 de HARP, ou un peptide dérivé de l'un de ceux-ci, éventuellement en association avec un peptide 111-136 de HARP ou un peptide dérivé, pour la préparation d'un médicament destiné au traitement d'une pathologie associée à une angiogénèse.

L'invention divulgue en outre une méthode pour inhiber l'angiogénèse ou pour traiter une pathologie associée à une angiogénèse, chez un mammifère, plus particulièrement un humain, comprenant l'administration chez ce mammifère d'une quantité thérapeutiquement efficace d'un acide nucléique comprenant une séquence codant pour un peptide 13-39, ou 65-97 de HARP, ou un peptide dérivé de l'un de ceux-ci, éventuellement en association avec un acide nucléique comprenant une séquence codant pour un peptide 111-136 de HARP ou un peptide dérivé de celui-ci.

### FIGURES :

La figure 1 est un graphe représentant l'inhibition de la prolifération cellulaire induite par HARP. Des cellules NIH 3T3 sont stimulées ou non par 4 nM d'HARP en présence ou non de peptide 13-39 ou 65-97 utilisé à une concentration allant de 0,1 µM à 10 µM. Après 24 heures d'incubation, la prolifération cellulaire est estimée par mesure de la thymidine tritiée incorporée par les cellules.
La figure 2 est un graphe qui représente l'inhibition de la croissance tumorale dans un modèle de souris nude ayant reçu une injection de cellules PC3 (lignée issue de cancer de prostate).
   Deux millions de cellules PC3 sont injectées à des lots de 5 souris nude traitées ou non par injection quotidienne au niveau de la tumeur par du PBS (contrôle) ou par le peptide HARP 111-136 à une concentration de 5 mg/kg. La croissance tumorale pour chaque lot d'animaux est évaluée par mesure de la taille de la tumeur au jour 9, 13, 16, 20, 23 et 27.

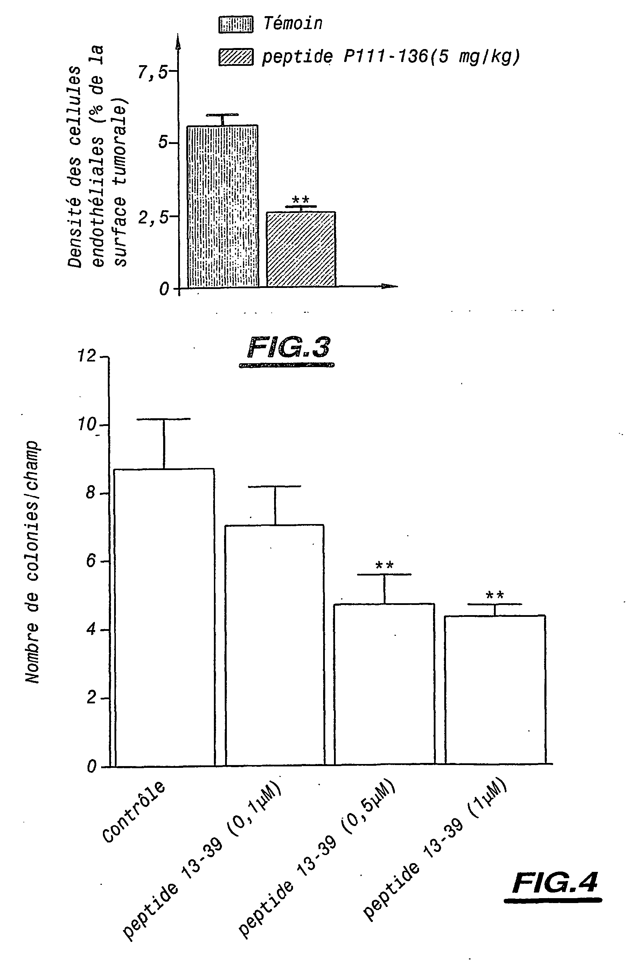

La figure 3 est un graphe représentant l'inhibition de l'angiogénèse tumorale dans un modèle de souris nude qui ont reçu une injection de cellules PC3 (lignée issue de cancer de prostate). Deux millions de cellules PC3 sont injectées à des lots de souris traitées ou non par injection quotidienne au niveau de la tumeur par du PBS (contrôle) ou par le peptide P111-136 à une concentration de 5 mg/kg. Les souris sont sacrifiées au jour 27. La quantification de l'angiogénèse est effectuée sur coupe après analyse immunohistologique en utilisant l'isolectine Bandeiraea Simplicifolia comme marqueur spécifique des cellules endothéliales et analyse d'images. Les valeurs présentées dans l'histogramme représentent la valeur moyenne obtenue en analysant trois coupes indépendantes issues de chacune des six tumeurs obtenues dans chaque condition.
La figure 4 est un graphe qui représente l'inhibition de la croissance des cellules tumorales MDA-MB-231 induite par le peptide HARP 13-39. Les cellules MDA-MB-231 sont ensemencées dans une boîte de culture de 12 puits contenant 1 ml d'agar à 0,6% en présence ou non de peptide HARP 13-39. Après 13 jours de culture les colonies ayant un diamètre égal ou supérieur à 50 µm sont comptées. Les résultats du graphe représentent les valeurs d'une expérience répétée 2 fois avec des valeurs similaires et chaque mesure est faite en triple. Les barres représentent les standards d'erreurs (*, p < 0.05; **, p < 0.01).

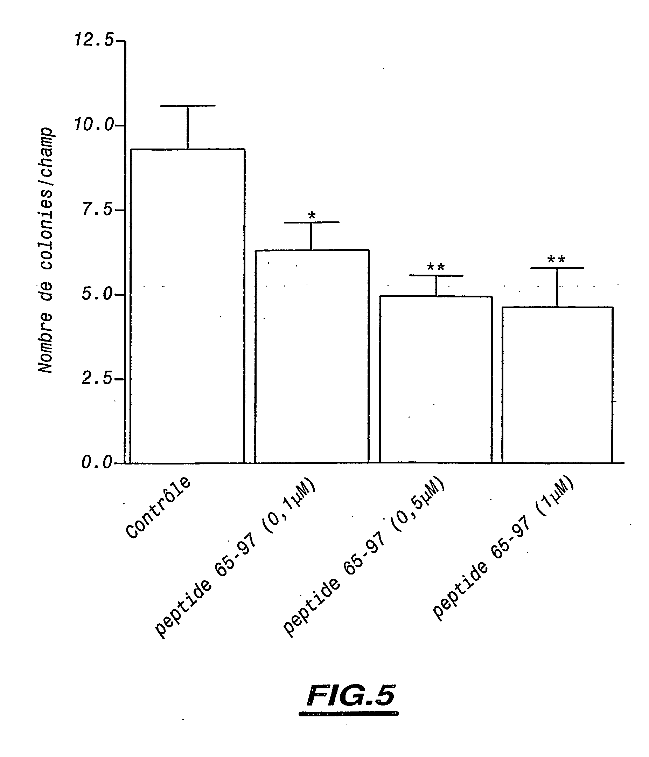

La figure 5 est un graphe qui représente l'inhibition de la croissance des cellules tumorales MDA-MB-231 induite par le peptide HARP 65-97. Les cellules MDA-MB-231 sont ensemencées dans une boîte de culture de 12 puits contenant 1 ml d'agar à 0,6% en présence ou non de peptide HARP 65-97. Après 13 jours de culture les colonies ayant un diamètre égal ou supérieur à 50 µm sont comptées. Les résultats du graphe représentent les valeurs d'une expérience répétée 2 fois avec des valeurs similaires et chaque mesure est faite en triple. Les barres représentent les standards d'erreurs (*, p < 0.05; **, p < 0.01).

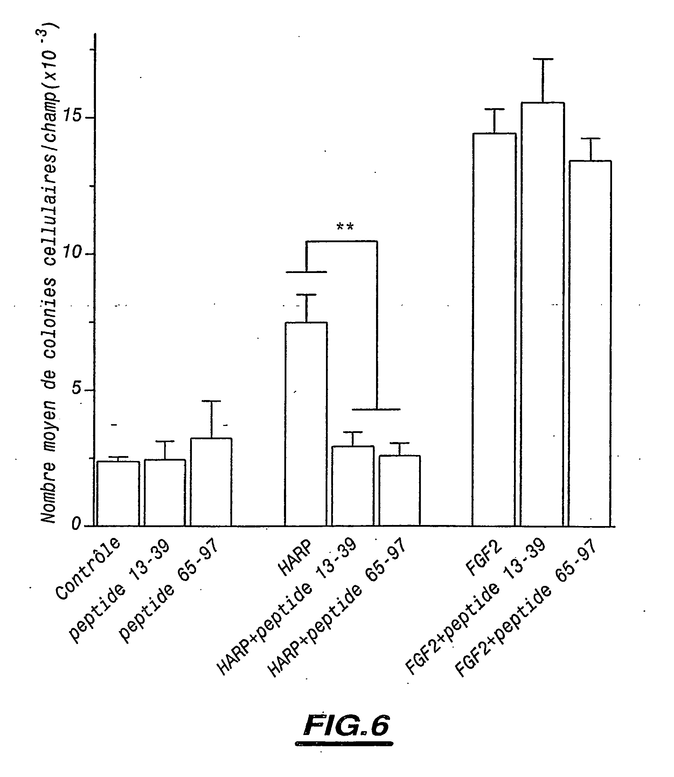

La figure 6 représente l'inhibition par les peptides HARP 13-39 et 65-97 de l'angiogénèse induite par l'HARP dans un modèle *in vivo.* Trois cents microlitres de Matrigel^{®} incubés avec de 5 nM d'HARP en présence ou non des peptides HARP 13-39 et 65-97 utilisés à une concentration de 1 µM sont injectés en sous cutané à une souris. Le contrôle de l'expérience est réalisé en utilisant du FGF-2. Sept jours plus tard, les souris sont sacrifiées et le Matrigel® est prélevé. Des coupes d'une épaisseur de 8 µM sont réalisées. Les coupes sont ensuite colorées au Trichrome de Gomori. Le nombres de cellules ayant envahi le Matrigel® est quantifié par analyse d'image avec le logiciel NIH. Les résultats représentent la valeur moyenne d'une analyse de 7 coupes par souris (n = 4 souris/groupe). Les barres représentent les standards d'erreurs (**, p < 0.01).

### EXEMPLES :

### Exemple 1 : Effet des peptides HARP 13-39 et 65-97 sur l'inhibition de la prolifération des cellules NIH 3T3 stimulées par HARP

Des cellules fibroblastiques de type NIH 3T3 sont ensemencées à une densité de 3 x 10⁴ cellules par cm² dans du milieu de culture DMEM supplémenté avec 10% de sérum de veau foetal. Après 24 heures d'incubation à 37°C dans une atmosphère contenant 7 % de CO₂, le milieu de culture est remplacé par du DMEM ne contenant pas de sérum de veau foetal. Vingt-quatre heures après, la molécule HARP (4 nM) en présence ou non des peptides HARP 16-48 ou 65-97 de séquences respectives SEQ ID N° 2 et N° 3 à une concentration allant de 0,1 à 10 µM est ajoutée pendant 18 heures. Après cette période d'incubation, 0,5 µCi of [*methyl*-³H]thymidine est ajouté et 6 heures après, les cellules sont fixées par une solution de 10% d'acide trichloracétique. La radioactivité incorporée par les cellules est alors comptée par scintillation liquide après avoir effectué une lyse cellulaire avec une solution de soude à une concentration de 0,1 N.

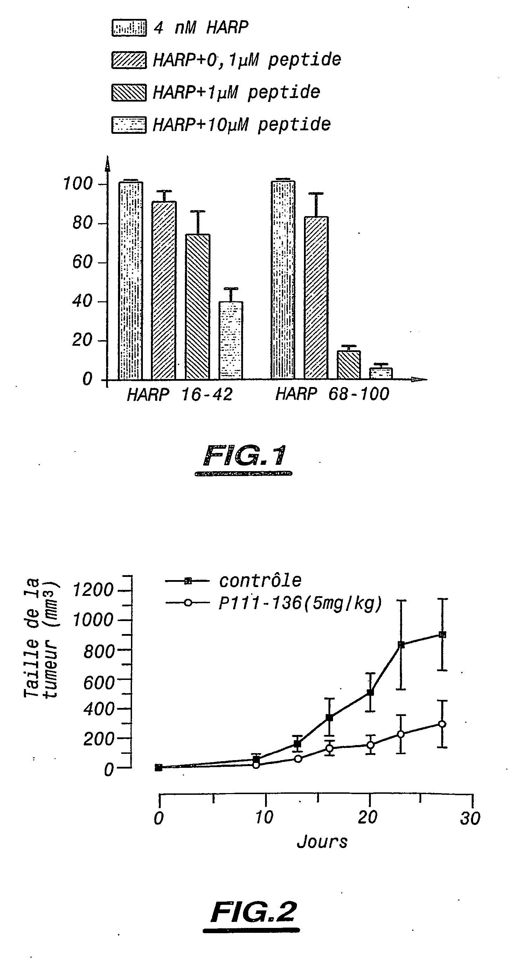

Les résultats sont présentés sur la figure 1 et montrent que les peptides correspondant à la séquence 13-39 et 65-97 sont capables d'inhiber d'une façon dose dépendante l'activité d'induction de la prolifération cellulaire d'HARP. A une dose de 10 µM, le peptide 65-97 inhibe plus de 90% de l'activité d'HARP, alors que l'on observe 60% d'inhibition pour le peptide 13-39.

### Exemple 2 : Effet du peptide HARP 111-136 sur l'inhibition de l'angiogénèse tumorale et de la croissance tumorale

La capacité du peptide P111-136 (SEQ ID N° 4) d'inhiber l'angiogénèse tumorale a été testée en induisant une croissance tumorale par injection de cellules PC3 à des souris nude en présence ou non de peptide P111-136. On injecte 2x10⁶ cellules PC3 à des lots de 5 souris nude (nude/nude, Laboratoire IFFA CREDO) traités ou non par injection au niveau de la tumeur de 100 µl par jour d'une solution de PBS (lot contrôle) ou par une solution de peptide P111-136 diluée dans du PBS à une concentration de 5 mg/kg. Au jour 9, 13, 16, 20, 23 et 27, la taille de la tumeur est mesurée à l'aide d'un pied à coulisse. Les résultats sont présentés à la figure 3 et indiquent que le peptide HARP 111-136 utilisé à une dose de 5 mg par kg induit une inhibition de la croissance des tumeurs.

L'analyse des données indique que la vitesse de croissance tumorale est 4 fois moins grande pour les souris traitées avec 5 mg/kg de peptide HARP 111-136 que celles non traitées. Au jour 27, on observe que pour le groupe de souris traitées, la taille moyenne des tumeurs est 80% moins grosse que chez celles n'ayant pas reçu de traitement.

Au jour 27, les tumeurs sont prélevées et des coupes à congélation de 5 µm sont réalisées. Après fixation des tissus à l'acétone (4°C, 15 min) et réhydratation de celles-ci, la présence des cellules endothéliales est révélée à l'aide de la lectine Bandeiraea Simplicifolia Isolectine B4 (10 µg/ml) qui se fixe spécifiquement à la surface des cellules endothéliales. Cette lectine est alors mise en évidence par immuno-marquage à l'aide d'un sandwich d'anticorps spécifiques marqués à la phosphatase alcaline. Après incubation avec un substrat de la phosphatase alcaline (Vector red substrat), les cellules endothéliales sont colorées en rouge. La quantification du nombre de cellules endothéliales dans la tumeur est réalisée par analyse d'image (NIH image) en analysant les 6 tumeurs sur 3 coupes indépendantes d'une même tumeur.

On observe un nombre de cellules endothéliales moins important pour les tumeurs traitées par le peptide que pour les tumeurs non traitées. La quantification de cet effet est montrée sur la figure 3. L'analyse de l'histogramme indique que l'injection du peptide P111-136 induit une diminution des vaisseaux intra-tumoraux de 55% démontrant que le peptide P111-136 est capable d'inhiber l'angiogénèse tumorale.

### Exemple 3 : Effet des peptides HARP 13-39 et 65-97 sur l'inhibition de la croissance sur agar mou des cellules tumorales

Des cellules MDA-MB-231 issues de carcinome mammaire humain, sont ensemencées à une densité de 3x10³ cellules par cm² dans du milieu de culture DMEM contenant 10% de sérum de veau foetal, 0.35% d'agar et contenant ou non des concentration de peptides HARP 13-39 et 65-97 (résidus 13 à 39 de SEQ ID N°1 et N°3). Les cellules sont ensemencées dans une boite de culture de 12 puits (35 mm de diamètre/puits) préalablement couverte par 1 ml d'agar à 0.6%. Les peptides sont ajoutés dans le milieu de culture tous le 2 jours, Après 13. jours d'incubation dans une atmosphère humide à 37°C et 7% CO2, les colonies ayant un diamètre égal ou supérieur à 50 µm sont comptées. Chaque point d'expérience est réalisé en triple et chaque expérience est répétée trois fois.

Les résultats représentés sur les figures 4 et 5 montrent que les peptides HARP 13-39 et 65-97 inhibent la croissance des cellules tumorales de façon dose dépendante. Ainsi, une inhibition de la croissance des cellules tumorales MDA-MB-231 de 53% est observée en présence de 1 µM de peptide HARP 13-39 (Fig. 4) et 41% en présence HARP 65-97 utilisé à la même concentration (Fig. 5).

### Exemple 4 : inhibition par les peptides 13-39 et 65-97 de l'angiogénèse induite par HARP

HARP étant un facteur de croissance impliqué dans la prolifération et la différenciation des cellules endothéliales, les inventeurs ont, dans l'expérience présentée ci-dessous, étudié l'effet des peptides HARP 13-39 et 65-97 (résidus 13 à 39 de SEQ ID N°1 et SEQ ID N°3) sur l'activité angiogène induite par HARP.

Cette expérience a été réalisée en utilisant un modèle d'angiogénèse *in vivo* chez la souris. Ce modèle expérimental consiste à injecter du Matrigel® contenant une substance à analyser pour ses propriétés stimulantes ou inhibitrices de l'angiogénèse. Quatre souris Swiss (Janvier, Le Genest St Isle, France) par groupe sont injectées avec 300 µl de Matrigel® préalablement incubé avec soit 5 nM d'HARP, 1 µM de peptide HARP 13-39 ou HARP 65-97, soit un mélange d'HARP (5 nM) et de peptides HARP 13-39 ou HARP 65-97 (1 µM). Le contrôle de la spécificité de l'inhibition est réalisé en utilisant un autre facteur angiogène, le FGF-2, testé soit seul à une concentration de 10 nM ou supplémenté des peptides HARP 13-39 ou HARP 65-97 utilisés à une concentration de 1 µM.

Les souris sont sacrifiées sept jours après l'injection du Matrigel®. Celui-ci est prélevé et congelé dans l'azote liquide. Des coupes de 8 µM d'épaisseur sont alors effectuées avec un cryostat. Les coupes sont ensuite colorées au Trichrome de Gomori. Le nombre de cellules endothéliales ayant envahi le Matrigel® est quantifié par analyse d'images en utilisant le logiciel NIH image.

Les résultats présentés à la figure 6 indiquent que l'HARP ainsi que le FGF-2 induisent une stimulation de l'angiogénèse. En comparaison au Matrigel® utilisé seul, une augmentation du nombre de cellules endothéliales d'un facteur 3 est observée pour HARP et de 5,8 fois pour le FGF-2. Utilisés à une concentration de 1 µM, les peptides HARP 13-39 ou HARP 65-97 inhibent totalement l'angiogénèse induite par l'HARP alors qu'ils n'ont aucun effet sur l'angiogenèse induite par le FGF-2. Aucun effet significatif n'est observé lorsque les peptides HARP 13-39 ou HARP 65-97 sont utilisés seuls.

Ces résultats montrent que les peptides HARP 13-39 ou HARP 65-97 sont des inhibiteurs spécifiques de l'activité angiogène induite par l'HARP,

### REFERENCES

- Bernard-Pierrot et al. (2001) J Biol Chem 276, 12228-12234
- Bernard-Pierrot et al. (2002) J Biol Chem 277(35) :32071-7
- Chauhan et al. (1993) Proc Natl Acad Sci U S A 90, 679-682
- Choudhuri et al. (1997) Cancer Res 57, 1814-1819
- Courty et al. (1991) Biochem Biophys Res Commun 180, 145-151
- Fang et al. (1992) J Biol Chem 267, 25889-25897
- Firat et al., (2002), J. Gen. Ther. 4:38-45
- Folkman, J. (1971) N Engl J Med 285, 1182-1186
- Furth et al., (1992) analytical Biochemistry, 205:365-368
- Jackson et al., (1988) Ann. Rheum. Dis. 57(3) :158-161)
- Jager et al. (1997) Int J Cancer 73, 537-543
- Huttunen et al. (2002), Cancer Research 62:4805-4811
- Kojima et al. (1995a) Biochem Biophys Res Commun 216, 574-581
- Kojima et al. (1995b) Biochem Biophys Res Commun 206, 468-473
- Ledoux et al. (1997) J Histochem Cytochem 45, 1239-1245
- Matsubara et al. (1990) J Biol Chem 265, 9441-9443
- Milhiet et al. (1998) J Endocrinol 158, 389-399
- Novotny et al. (1993) Arterioscler Thromb 13, 1798-1805
- Papadimitriou et al. (2000) Biochem Biophys Res Commun 274, 242-248
- Papadimitriou et al. (2001) Biochem Biophys Res Commun 282, 306-313
- Rauvala, H. (1989) Embo J 8, 2933-2941
- Riegel et al. (1994) Breast Cancer Res Treat 31, 309-314
- Robinson et al., (1997) Immunology 9 :271-83
- Sambrook (1989) Cold Spring Harbor Laboratory Press, Cold Spring Harbor, New York
- Stoica et al. (2001) J Biol Chem 276, 16772-16779
- Taguchi et al. (2000) Nature 405 :354-360
- Tang et al., (1992) Nature 356, 152-154
- Tsutsui et al. (1991) Biochem Biophys Res Commun 176, 792-797
- Vacherot et al. (1999a) Prostate 38, 126-136
- Vacherot et al. (1999b) J Biol Chem 274, 7741-7747
- Wellstein et al. (1992) J Biol Chem 267, 2582-2587
- Yeh et al. (1998) J Neurosci 18, 3699-3707

## Revendications

1. Peptide consistant en la séquence d'acides aminés choisie parmi :
- la séquence 13-39 du facteur HARP (résidus 13 à 39 de SEQ ID NO: 1); et
- la séquence 65-97 du facteur HARP (SEQ ID NO: 3).

2. Peptide selon la revendication 1, **caractérisé en ce que** ledit peptide consiste en les résidus 13 à 39 de la séquence SEQ ID NO: 1.

3. Peptide consistant en une séquence d'acides aminés au moins 80 % identique à la séquence SEQ ID NO: 2 ou NO: 3, et présentant une activité d'inhibition de l'angiogénèse, et une capacité de se fixer aux glycoaminoglycanes (GAG).

4. Peptide selon la revendication 3, dont la séquence diffère de la séquence SEQ ID NO: 2 ou NO: 3 par la substitution conservative d'au moins un acide aminé.

5. Acide nucléique consistant en une séquence codant pour un peptide tel que défini à l'une des revendications 1 à 4.

6. Acide nucléique selon la revendication 5, consistant en la séquence SEQ ID NO: 5 ou SEQ ID NO: 6.

7. Procédé de production d'un peptide tel que défini à l'une des revendications 1 à 4, comprenant la synthèse dudit peptide par voie chimique.

8. Procédé de production d'un peptide tel que défini à l'une des revendications 1 à 4, dans lequel un vecteur contenant un acide nucléique tel que défini à l'une des revendications 5 ou 6 est transféré dans une cellule hôte qui est mise en culture dans des conditions permettant l'expression du peptide correspondant.

9. Composition pharmaceutique comprenant un peptide tel que défini à l'une des revendications 1 à 4, et un ou plusieurs excipients pharmaceutiquement acceptables.

10. Composition selon la revendication 9, comprenant en outre un peptide ayant la séquence d'acides aminés 111-136 du facteur HARP ou à un peptide consistant en une séquence d'acides aminés au moins 80 % identique à la séquence SEQ ID NO: 4, et présentant une activité d'inhibition de l'angiogénèse, et une capacité de se fixer au récepteur ALK.

11. Composition selon la revendication 10, comprenant :
- le peptide consistant en les résidus 13 à 39 de la séquence SEQ ID NO: 1 ;
- le peptide 65-97 de séquence SEQ ID NO: 3 ; et
- le peptide 111-136 de séquence SEQ ID NO: 4.

12. Composition pharmaceutique comprenant un acide nucléique consistant en une séquence codant pour un peptide tel que défini à l'une des revendications 1 à 4.

13. Composition selon la revendication 12, comprenant en outre un acide nucléique comprenant une séquence codant pour un peptide tel que défini à la revendication 10.

14. Composition selon la revendication 13, comprenant :
- un acide nucléique codant pour le peptide consistant en les résidus 13-39 de la séquence SEQ ID NO: 1O: ;
- un acide nucléique codant pour le peptide 65-97 de séquence SEQ ID NO: 3 ;
- un acide nucléique codant pour le peptide 111-136 de séquence SEQ ID NO: 4.

15. Composition selon la revendication 13 ou 14, dans laquelle lesdites acides nucléiques sont portés par un unique vecteur.

16. Peptide tel que défini à l'une des revendications 1 à 4, pour utilisation dans le traitement d'une pathologie associée à une angiogénèse choisie parmi une tumeur, une lésion oculaire, la polyarthrite rhumatoïde et une maladie de peau.

17. Peptide selon la revendication 16, dans laquelle le peptide tel que défini à l'une des revendications 1 à 4 est associé à un peptide ayant la séquence d'acides aminés 111-136 du facteur HARP ou à un peptide consistant en une séquence d'acides aminés au moins 80 % identique à la séquence SEQ ID NO: 4, et présentant une activité d'inhibition de l'angiogénèse, et une capacité de se fixer au récepteur ALK.

18. Acide nucléique tel que défini à l'une des revendications 5 ou 6, pour utilisation dans le traitement d'une pathologie associée à une angiogénèse choisie parmi une tumeur, une lésion oculaire, la polyarthrite rhumatoïde et une maladie de peau.

19. Acide nucléique selon la revendication 18, dans lequel l'acide nucléique tel que défini à l'une des revendications 5 ou 6 est associé à un acide nucléique comprenant une séquence codant pour un peptide tel que défini à la revendication 10.

## Claims

1. Peptide consisting in the sequence of amino acids selected from:
- the sequence 13-39 of the HARP factor (residues 13 to 39 of SEQ ID NO: 1); and
- the sequence 65-97 of the HARP factor (SEQ ID NO: 3).

2. Peptide according to claim 1, **characterized in that** said peptide consists in the residues 13 to 39 of the sequence SEQ ID NO: 1.

3. Peptide consisting in a sequence of amino acids at least 80 % identical to the sequence SEQ ID NO: 2 or NO: 3, and exhibiting an angiogenesis inhibiting activity and a capacity for binding to glycoaminoglycans (GAG).

4. Peptide according to claim 3, in which the sequence differs from the sequence SEQ ID NO: 2 or NO: 3 by the conservative substitution of at least one amino acid.

5. Nucleic acid consisting in a sequence coding for a peptide as defined in any one of claims 1 to 4.

6. Nucleic acid according to claim 5, consisting in the sequence SEQ ID NO: 5 or SEQ ID NO: 6.

7. Method of production of a peptide as defined in any one of claims 1 to 4, comprising the synthesis of the said peptide by chemical means.

8. Method of production of a peptide as defined in any one of claims 1 to 4, in which a vector containing a nucleic acid as defined in claim 5 or 6 is transferred into a host cell which is cultured under conditions permitting the expression of the corresponding peptide.

9. Pharmaceutical composition comprising a peptide as defined in any one of claims 1 to 4, and one or more pharmaceutically acceptable excipients.

10. Composition according to claim 9, further comprising a peptide having the sequence of amino acids 111-136 of the HARP factor or a peptide consisting in a sequence of amino acids at least 80 % identical to the sequence SEQ ID NO: 4, and exhibiting an angiogenesis inhibiting activity and a capacity for binding to the ALK receptor.

11. Composition according to claim 10, comprising:
- the peptide consisting in the residues 13 to 39 of the sequence SEQ ID NO: 1;
- the peptide 65-97 of sequence SEQ ID NO: 3 ; et
- the peptide 111-136 of sequence SEQ ID NO: 4.

12. Pharmaceutical composition comprising a nucleic acid consisting in a sequence coding for a peptide as defined in any one of claims 1 to 4.

13. Composition according to claim 12, further comprising a nucleic acid comprising a sequence coding for a peptide as defined in claim 10.

14. Composition according to claim 13, comprising:
- a nucleic acid coding for the peptide consisting in the residues 13-39 of the sequence SEQ ID NO: 1O ;
- a nucleic acid coding for the peptide 65-97 of sequence SEQ ID NO: 3 ;
- a nucleic acid coding for the peptide 111-136 of sequence SEQ ID NO: 4.

15. Composition according to claim 13 or 14, in which the said nucleic acids are carried by one single vector.

16. Peptide as defined in any one of claims 1 to 4, for use in the treatment of a pathology associated with an angiogenesis selected from a tumour, an ocular lesion, rheumatoid polyarthritis and a skin disease.

17. Peptide according to claim 16, in which the peptide as defined in any one of claims 1 to 4 is associated with a peptide having the sequence of amino acids 111-136 of the HARP factor or with a peptide consisting in a sequence of amino acids at least 80 % identical to the sequence SEQ ID NO: 4, and exhibiting an angiogenesis inhibiting activity and a capacity for binding to the ALK receptor.

18. Nucleic acid as defined in claim 5 or 6, for use in the treatment of a pathology associated with an angiogenesis selected from a tumour, an ocular lesion, rheumatoid polyarthritis and a skin disease.

19. Nucleic acid according to claim 18, in which the nucleic acid as defined in claim 5 or 6 is associated with a nucleic acid comprising a sequence coding for a peptide as defined in claim 10.

## Patentansprüche

1. Peptid, bestehend aus einer Aminosäuresequenz, ausgewählt unter:
- der Sequenz 13-39 des HARP-Faktors (Reste 13 bis 39 von SEQ ID NO: 1) und
- der Sequenz 65-97 des HARP-Faktors (SEQ ID NO: 3).

2. Peptid nach Patentanspruch 1, **dadurch gekennzeichnet, dass** das genannte Peptid aus den Resten 13 bis 39 der Sequenz SEQ ID NO: 1 besteht.

3. Peptid, bestehend aus einer Aminosäuresequenz, die zu mindestens 80% mit der Sequenz SEQ ID NO: 2 oder NO: 3 identisch ist und eine antiangiogene Aktivität aufweist, sowie die Fähigkeit, sich an Glykoaminoglykane (GAG) zu binden.

4. Peptid nach Patentanspruch 3, dessen Sequenz sich von der Sequenz SEQ ID NO: 2 oder NO: 3 in der konservativen Substitution mindestens einer Aminosäure unterscheidet

5. Nukleinsäure, bestehend aus einer Sequenz, die für ein in einem der Patentansprüche 1 bis 4 definiertes Peptid kodiert.

6. Nukleinsäure nach Patentanspruch 5, bestehend aus der Sequenz SEQ NO: 5 oder SEQ ID NO: 6.

7. Verfahren zur Herstellung eines in einem der Patentansprüche 1 bis 4 definierten Peptids, die Synthese des genannten Peptids auf chemischem Wege umfassend.

8. Verfahren zur Herstellung eines in einem der Patentansprüche 1 bis 4 definierten Peptids, in dem ein Vektor, der eine in einem der Patentansprüche 5 oder 6 definierte Nukleinsäure enthält, in eine Wirtszelle übertragen wird, die unter Bedingungen kultiviert wird, die eine Expression des entsprechenden Peptids erlauben.

9. Pharmazeutisches Präparat, ein in einem der Patentansprüche 1 bis 4 definiertes Peptid enthaltend und ein oder mehrere pharmazeutisch geeignete Bindemittel.

10. Pharmazeutisches Präparat nach Patentanspruch 9, außerdem ein Peptid enthaltend, das die Aminosäuresequenz 111-136 des HARP-Faktors aufweist oder ein Peptid, das aus einer Aminosäuresequenz besteht, die zu mindestens 80% mit der Sequenz SEQ ID NO: 4 identisch ist und eine antiangiogene Aktivität aufweist und die Fähigkeit, sich an den Rezeptor ALK zu binden.

11. Präparat nach Patentanspruch 10, umfassend:
- das aus den Resten 13 bis 39 der Sequenz SEQ ID NO: 1 bestehende Peptid,
- das Peptid 65-97 der Sequenz SEQ ID NO: 3, und
- das Peptid 111-136 der Sequenz SEQ ID NO: 4.

12. Pharmazeutisches Präparat, eine Nukleinsäure umfassend, die aus einer Sequenz besteht, die für ein in einem der Patentansprüche 1 bis 4 definiertes Peptid kodiert.

13. Präparat nach Patentanspruch 12, außerdem eine Nukleinsäure umfassend, die aus einer Sequenz besteht, die für ein in Patentanspruch 10 definiertes Peptid kodiert.

14. Präparat nach Patentanspruch 13, umfassend:
- eine Nukleinsäure, die für das aus den Resten 13 bis 39 der Sequenz SEQ ID NO: 10: bestehende Peptid kodiert,
- eine Nukleinsäure, die für das Peptid 65-97 der Sequenz SEQ ID NO: 3 kodiert, und
- eine Nukleinsäure, die für das Peptid 111-136 der Sequenz SEQ ID NO: 4 kodiert.

15. Präparat nach Patentanspruch 13 oder 14, in dem die genannten Nukleinsäuren von einem einzigen Vektor getragen werden.

16. Peptid, wie in einem der Patentansprüche 1 bis 4 definiert, zur Anwendung in der Behandlung einer Pathologie, die mit einer Angiogenese verbunden ist, ausgewählt unter Tumoren, Augenverletzungen, rheumatoide Polyarthritis und Hautkrankheiten.

17. Peptid nach Patentanspruch 16, in dem das in einem der Patentansprüche 1 bis 4 definierte Peptid mit einem Peptid verbunden ist, das die Aminosäuresequenz 111-136 des HARP-Faktors aufweist, oder mit einem Peptid, bestehend aus einer Aminosäuresequenz, die zu mindestens 80% mit der Sequenz SEQ ID NO: 4 identisch ist, und über antiangiogene Aktivität verfügt und die Fähigkeit, sich an den Rezeptor ALK zu binden.

18. Nukleinsäure, wie in einem der Patentansprüche 5 oder 6 definiert, zur Anwendung in der Behandlung einer Pathologie, die mit einer Angiogenese verbunden ist, ausgewählt unter Tumoren, Augenverletzungen, rheumatoide Polyarthritis und Hautkrankheiten.

19. Nukleinsäure nach Patentanspruch 18, in der die in einem der Patentansprüche 5 oder 6 definierte Nukleinsäure mit einer Nukleinsäure verbunden ist, die eine Sequenz aufweist, die für ein in Patentanspruch 10 definiertes Peptid kodiert.
